# EUROPEAN PATENT SPECIFICATION

(11) **EP 1 495 767 B1**
(45) Date of publication and mention of the grant of the patent: **25.10.2006**
(21) Application number: 03746173.8
(22) Date of filing: 15.04.2003
(51) Int. Cl.: A61K 39/39, A61K 39/00, A61P 35/00, A61P 37/04

(54) **IMMUNOADJUVANT**
IMMUNOADJUVANS
IMMUNOADJUVANT

(30) Priority: 18.04.2002 JP 2002116132
(43) Date of publication of application: 12.01.2005
(73) Proprietor: RIKEN, Wako-shi, Saitama 351-0198 (JP); Cell-Medicine, Inc., Ushiku-shi, Ibaraki 300-1234 (JP)
(72) Inventor: OHNO, Tadao, Ushiku-shi, Ibaraki 300-1234 (JP); UCHIMURA, Eiji c/o RIKEN, TSUKUBA INSTITUTE, Tsukuba-shi, Ibaraki 305-0074 (JP); HUANG, Lan c/o RIKEN, TSUKUBA INSTITUTE, Tsukuba-shi, Ibaraki 305-0074 (JP); KUANG, Ming c/o RIKEN, TSUKUBA INSTITUTE, Tsukuba-shi, Ibaraki 305-0074 (JP)
(74) Representative: Sternagel, Fleischer, Godemeyer & Partner
(86) International application number: PCT/JP2003/004767
(87) International publication number: WO 2003/086455

(56) References cited:
- EP-A1- 1 159 967
- PENG BAO GANG ET AL: "Autologous fixed tumor vaccine: a formulation with cytokine-microparticles for protective immunity against recurrence of human hepatocellular carcinoma." JAPANESE JOURNAL OF CANCER RESEARCH : GANN. APR 2002, vol. 93, no. 4, 30 April 2002 (2002-04-30), pages 363-368, XP002322082 ISSN: 0910-5050
- LIU S.Q. ET AL.: 'Adjuvant effect of GM-CSF-HSA/heparin microparticles in cancer immunotherapy' PROCEEDINGS OF THE INTERNATIONAL SYMPOSIUM ON CONTROLLED RELEASE OF BIOACTIVE MATERIALS vol. 26, 1999, pages 136 - 137, XP002971099
- WILKINSON KATALIN A. ET AL.: 'Enhancement of the human T cell response to culture filtrate fractions of mycobacterium tuberculosis by microspheres' JURNAL OF IMMUNOLOGICAL METHODS vol. 235, 2000, pages 1 - 9, XP004188226
- NEIDHART J.A. ET AL.: 'Active specific immunotherapy of stage IV renal carcinoma with aggregated tumor antigen adjuvant' CANCER vol. 46, no. 5, 01 September 1980, pages 1128 - 1134, XP002971100
- TIMMERMAN J.M. ET AL.: 'Linkage of foreign carrier protein to a self-tumor antigen enhances the immunogenicity of a pulsed dendritic cell vaccine' J. IMMUNOL. vol. 164, no. 9, 01 May 2000, pages 4797 - 4803, XP002972101
- ELIAS E.G. ET AL.: 'Adjuvant immunotherapy in melanoma: a new approach' J. SURG. ONCOL. vol. 50, no. 3, July 1992, pages 144 - 148, XP002972102
- GOLUMBEK P.T. ET AL.: 'Controlled release, biodegradable cytokine depots: a new approach in cancer vaccine design' CANCER RES. vol. 53, no. 24, 15 December 1993, pages 5841 - 5844, XP000651758

## Description

### Technical Field

The present invention relates to an immunoadjuvant.

### Background Art

Tuberculin has long been used as a test reagent for detecting an anamnestic infection with Mycobacterium tuberculosis or that for detecting positive conversion after administration of *Mycobacterium bovis* BCG (hereinafter abbreviated as "BCG"). A purified tuberculin obtained by purifying protein components in crude tuberculin through ammonium sulfate precipitation is extremely safe, because no hypersensitive inflammatory responses of skin is induced even when the tuberculin itself is repeatedly administered to human, although some booster effect is observed in the so-called tuberculin reaction test (Singh, D. et al., Am. J. Respir. Crit. Care Med. 2001, Sep 15; 164(6):962-4).

The inventors of the present invention found that antitumor immunoreactions against tumor cells can be efficiently induced by administration of a solidified or microparticulated tumor tissue to a body together with at least one kind of cytokine or cytokine inducer (PCT/JP00/00692). They also found that potent antitumor immunoreaction against tumor cells can be induced in the above process by simultaneous administration of a purified tuberculin in a soluble form as a general immunoadjuvant (PCT/JP00/0692). As described above, components of tuberculin can be utilized as an immunoadjuvant. However, since tuberculin components are soluble, they have a drawback that they rapidly disappear from the site of administration by diffusion.

It is known that components in a filtrate of a culture of *M. tuberculosis* promote T cell reactions after being bound to polystyrene microparticles, although they have only weak adjuvant activity in a dissolved state(Wilkinson, K. A., et al., J. Immunol. Methods, 235:1-9, 2000). When an adjuvant in a dissolved state is immobilized on an insoluble adjuvant carrier, the adjuvant does not rapidly disappear by diffusion and thereby exhibits potent adjuvant activity. However, although the polystyrene microparticles in this immobilization product are phagocytized by antigen-presenting cells, they are not degraded in the cells and become undesirable plastics remaining in a body.

As a means to solve the aforementioned problem, a method is cited in said literature in which components in a filtrate of a culture of bacteria is bound to biodegradable synthetic polymer microparticles (Vordermeier, H.M., et al., Vaccine, 13, 1576-1582, 1995; Ertl, H.C., et al., Vaccine, 14, 879-885, 1996; Jones D.H., et al., J. Biotechnology, 44, 29-36, 1996; Venkataprasad, N., et al., Vaccine, 17, 1814-1819, 1999). However, it is known that the disclosed synthetic polymer, i.e., poly(DL-lactide co-glycolide) (PLG), generates lactic acid upon degradation, and results in acidification of a local environment where the degradation occurs, and thus this method is also undesirable for living bodies.

The European patent application EP 1 159 967 discloses immunoadjuvants consisiting of GM-CSF or IL-2 incorporated in heparin/human serum albumin-microspheres, which were used as vaccine in combination with fixed Hepa 1-6 cells or fixed tumor tissue, respectively, and the adjuvant Titer Max Gold or tuberculin, respectively. In the document the therapeutic efficiency of the vaccine is demonstrated. Further, methods for preparing microparticles prepared from solidified tumor material and methods of fixing soluble tumor antigens to solid microparticles are disclosed. The cells can be inactivated by physical means.

Accordingly, an adjuvant that is solid and biodegradable and free from the aforementioned undesirable actions has been desired in the field of tumor immunology. Further, among conventional techniques, no immunoadjuvant is available which can efficiently stimulate antitumor immunoreactions against live tumor cells when administered to a denatured tissue, which tissue is obtained beforehand by physical denaturation of a tumor tissue or tumor cells containing complex and diverse tumor antigens in the body of an individual of a syngeneic animal.

### Disclosure of the Invention

As mentioned above, tuberculin is extremely safe even when repeatedly administered to a human, although tuberculin has only weak adjuvant activity in a dissolved state. An object of the present invention is to provide, by using tuberculin, an immunoadjuvant which can efficiently exhibit potent adjuvant activity but avoid undesirable conditions for living bodies .

The inventors of the present invention conducted various researches to achieve the foregoing object. As a result, they found that potent adjuvant activity was successfully obtained by providing a sustained-release preparation of tuberculin. Further, during the study of the sustained-release preparation of tuberculin, the inventors of the present invention also found that, when albumin and heparin were mixed to form precipitates by coacervation, tuberculin proteins were taken into these precipitates, and thereby insoluble microparticles were formed, and that when these insoluble microparticles were administered to a body together with an antigen and soluble purified tuberculin, they exhibited potent tumor preventive effect, and when they were administered to a thermally denatured tumor tissue *in vivo,* antitumor immunoreactions were successfully induced. The present invention was achieved on the basis of these findings.

The present invention thus provides an immunoadjuvant, which comprises:
precipitates formed by coacervation of
   (a) a soluble protein (provided that a soluble protein contained in tuberculin is excluded), and
   (b) a mucopolysaccharide
      and further comprises:
   (c) a soluble protein contained in tuberculin
   wherein said (c) is coprecipitated with the precipitates.

According to preferred embodiments of the above invention, there are provided the aforementioned immunoadjuvant, wherein the soluble protein of the component (a) is albumin, and the mucopolysaccharide of the component (b) is heparin; the aforementioned immunoadjuvant, wherein the precipitates are crosslinked with an intermolecular crosslinking agent for proteins; the aforementioned immunoadjuvant, wherein the aforementioned component (c) consists of a combination of a soluble protein contained in tuberculin and a soluble protein having antigenicity; and the aforementioned immunoadjuvant, wherein the soluble protein having antigenicity is derived from a tumor tissue, tumor cell, tumor cell component, and/or tumor antigen peptide/tumor-associated antigen.

From another aspect, the present invention provides an immunoadjuvant in the form of an insoluble microparticle which comprises (c) a soluble protein contained in tuberculin and (d) an insoluble protein molecule, wherein the components (c) and (d) are crosslinked with an intermolecular crosslinking agent for proteins. According to a preferred embodiment of this invention, there is provided the aforementioned immunoadjuvant, wherein the insoluble protein molecule is collagen.

According to further embodiments, there are provided the aforementioned immunoadjuvant which is administered *in vivo* to a mammal including human after mixing with an antigen and is used for inducing a systemic immunoreaction against said antigen; and the aforementioned immunoadjuvant, wherein the antigen is derived from a tumor tissue, tumor cell, tumor cell component, and/or tumor antigen peptide/tumor-associated antigen.

The present invention further provides a tumor vaccine containing the aforementioned immunoadjuvant. According to preferred embodiments of this invention, there are provided the aforementioned tumor vaccine, which is for inducing an antitumor immunoreaction by administration to a tumor tissue denatured by a physical means; the aforementioned tumor vaccine, wherein the physical means is selected from a group consisting of microwave irradiation, radio frequency coagulation, freezing coagulation, electrosurgical knife heating, hot water injection, alcohol injection, embolization, radioactive ray irradiation, laser beam irradiation, and ultrasonic disruption; the aforementioned tumor vaccine, which is for stimulating an immunoreaction *in vivo* by administration after mixing with an immunocompetent cell outside the body; the aforementioned tumor vaccine, wherein the immunocompetent cell is selected from a group consisting dendritic cell, B lymphocyte, T lymphocyte, and natural killer cell; and the aforementioned tumor vaccine, which is for being mixed extracorporeally with an immunocompetent cell and an antigen and then administered.

Further, from still other aspects, there are provided a method for inducing a systemic immunoreaction, which comprises the step of administering the aforementioned immunoadjuvant to a mammal including human; a method for therapeutic treatment of a tumor, which comprises the step of administering the aforementioned tumor vaccine to a tumor tissue denatured by a physical means; a method for inducing an antitumor immunoreaction, which comprises the step of administering the aforementioned tumor vaccine to a tumor tissue denatured by a physical means; and a method for stimulating an immunoreaction *in vivo,* which comprises the steps of mixing the aforementioned tumor vaccine *ex vivo* with an immunocompetent cell, and then administering the mixture to a body of a mammal including human.

### Brief Description of Drawings

Fig. 1 shows suppressive effect of the tumor vaccine of the present invention against liver cancer proliferation. The dotted line indicates cumulative survival rate (control group) and the solid line indicates cumulative survival rate (group administered with the microparticulated tuberculin vaccine). The thin dotted line indicates that of positive control group.
Fig. 2 shows prevention effect of the tumor vaccine of the present invention against tumor recurrence which was administered to a tumor tissue subjected to microwave coagulation.

### Best Mode for Carrying out the Invention

The immunoadjuvant of the present invention is characterized by comprising precipitates formed by coacervation of:
(a) a soluble protein (except for a soluble protein contained in tuberculin), and
(b) a mucopolysaccharide, and
(c) a soluble protein contained in tuberculin
wherein said (c) is coprecipitated with the precipitates.

Combination of the aforementioned (a) soluble protein and (b) mucopolysaccharide is not particularly limited so long as both produce precipitates by coacervation under a condition well known to those skilled in the art. Technical explanation of coacervation is described in detail in, for example, U.S. Patent No. 5,759,582. However, this term should not be construed in any limitative sense, and should be construed in its broadest meaning. For example, albumin and heparin can be preferably used as (a) the soluble protein and the mucopolysaccharide, respectively, however, the combination of (a) the soluble protein and (b) the mucopolysaccharide is not limited to the aforementioned combination. As the soluble protein contained in tuberculin, for example, total proteins contained in purified tuberculin can be used, as well as all or a part of soluble proteins prepared from tuberculin by a method well known to those skilled in the art. By stirring the aforementioned three kinds of components, precipitates are formed by coacervation of the components (a) and (b), and in this process, the component (c) is taken into the precipitates and coprecipitated to produce precipitates.

A ratio of (c) the soluble protein contained in tuberculin and (a) the soluble protein is not particularly limited so long as the ratio is selected within a range that (a) the soluble protein and (b) the mucopolysaccharide will cause coacervation. For example, when a solution of 2.5% human serum albumin at pH 2.5 is used as a soluble protein, a commercially available heparin solution at approximately 5 mg/ml is used as the mucopolysaccharide, 2.5 *µ*g of a soluble protein of tuberculin is dissolved in 600 *µ*1 of the mucopolysaccharide, and the aforementioned human serum albumin solution is added dropwise to the mixture with stirring with different volume ratios of 1/1.75, 1/1.5, 1/1.25, and 1/1 to form microparticles by coacervation. It is preferable that each suspension is centrifuged and a protein content in the supernatant is quantified, and a volume ratio is chosen at which most of the mixed protein is taken into microparticles. It should be noted that the ratio of the components (a) to (c), conditions of coacervation and the like can be suitably selected by those skilled in the art. A particle size of the microparticles contained in the resulting precipitates is generally 1 *µ*m or less, but not limited to a particular size.

The resulting precipitates, preferably precipitates in the forms of microparticles, can be used as an immunoadjuvant without any treatment. If necessary, the precipitates may be washed with distilled water. Further, for stabilization of the precipitates, insoluble microparticles may be prepared by forming crosslinks under treatment with an intermolecular crosslinking agent for proteins. A type of the intermolecular crosslinking agent for proteins is not particularly limited, and any agent well known to those skilled in the art can be used in a well-known manner. For example, when a 20 mg/ml aqueous solution of 1-ethyl-3-(3-dimethylaminopropyl)carbodiimide is added to proteins at a final concentration of 0.8 to 1.5 mg/ml with stirring by using a vortex mixer, and then the mixture is left standing at room temperature for 15 minutes, sufficiently stable crosslinks are formed between protein molecules in the precipitates. However, formation of crosslinks is not limited to those applying the aforementioned particular condition and using the particular intermolecular crosslinking agent for proteins. The immunoadjuvant obtained as described above, preferably that subjected to a treatment for crosslinking, is not dissolved even when washed with water, and can be preferably used as the immunoadjuvant of the present invention.

Further, according to another preferred embodiment of the present invention, an immunoadjuvant containing a soluble protein having antigenicity and a soluble protein contained in tuberculin having adjuvant activity can be produced by obtaining precipitates in the same manner as described above, except that the soluble protein having antigenicity and the soluble protein contained in tuberculin is mixed beforehand, and then the resulting mixture is used instead of the above (c).

From another aspect, the present invention also provides an immunoadjuvant in the forms of insoluble microparticles comprising a soluble protein contained in tuberculin and an insoluble protein molecule both of which are crosslinked with an intermolecular crosslinking agent for proteins. As the insoluble protein molecule, biodegradable insoluble protein molecules can be used. For example, collagen can be used. However, the insoluble protein molecule is not necessarily limited to collagen, and any insoluble protein molecule that is degradable *in vivo* can be used. A type of the intermolecular crosslinking agent for proteins is not particularly limited, and any agent well known to those skilled in the art can be used in a well-known manner. For example, 1-ethyl-3-(3-dimethylaminopropyl)carbodiimide and the like can be used. In general, a particle size of microparticles is preferably 1 *µ*m or less, however, not limited to the above particular size.

The immunoadjuvant of the present invention can be used as a general immunoadjuvant. The immunoadjuvant may simply be mixed with an antigen and administered to a body of a human or an animal, thereby systemic immunoreactions against the antigen can be induced. When the antigen is derived from tumor tissue, tumor cell, tumor cell component, and/or tumor antigen peptide/tumor-associated antigen, the immunoadjuvant can be used as a tumor vaccine. For example, by mixing the immunoadjuvant of the present invention with cancer cells isolated from a patient and immobilized, and then the mixture is intracutaneously administered to the patient, antitumor immunoreactions against the cancer cells can be induced in the patient *in vivo.* However, the method of using the immunoadjuvant of the present invention is not limited to the aforementioned method, and any conventional method utilizing an immunoadjuvant may be applied.

Further, by denaturing a tumor tissue in a body of a patient by a physical means and then administering the immunoadjuvant of the present invention to the tissue, antitumor immunoreactions can be induced against tumor cells surviving in the body of the patient. The physical means for denaturing the tumor tissue is not particularly limited, and examples thereof include, for example, means of microwave irradiation, radio frequency coagulation, freezing coagulation, electrosurgical knife heating, hot water injection, alcohol injection, embolization, radioactive ray irradiation, laser beam irradiation, ultrasonic disruption and the like. However, the means are not limited to these examples, and any means can be used so long as the means can induce cell death of tumor cells in a tumor tissue. Two or more types of physical means may be appropriately used in combination.

For example, when a tumor tissue is coagulated with heating by microwave irradiation, and the immunoadjuvant of the present invention is administered into the coagulated tissue, antitumor immunoreactions can be induced against tumor cells surviving inside or surrounding the tumor tissue. When the immunoadjuvant of the present invention is administered, it is also preferable to administer a tuberculin solution at the same time. However, the method of administering the immunoadjuvant of the present invention is not limited to the aforementioned embodiments. Any method can be applied so long as the method can provide an environment in which the immunoadjuvant of the present invention is taken into antigen-presenting cells that migrate to the denatured tumor tissue together with the tumor antigens contained in the denatured tumor tissue, or the immunoadjuvant of the present invention can directly stimulate antigen-presenting cells.

Further, immunoreactions in the body can also be stimulated by extracorporeally mixing beforehand the immunoadjuvant of the present invention and immunocompetent cells, and then administering the mixture to a body of a patient. As the immunocompetent cells, dendritic cells, B lymphocytes, T lymphocytes, and/or natural killer cells or the like can be preferably used. However, the immunocompetent cells are not limited to these cells.

From a still further aspect, the present invention provides a vaccine comprising the aforementioned immunoadjuvant as an active ingredient. When this vaccine is administered, immunocompetent cells may also be mixed. Further, a tumor tissue and/or tumor cells can be used as antigens by mixing with the vaccine. By administration of a vaccine obtained as described above to a patient from whom the tumor is derived, the tumor can be therapeutically treated.

### Examples

The present invention will be explained more specifically with reference to the following examples. However, the scope of the present invention is not limited to these examples. The immunoadjuvant of the present invention prepared in these examples may be referred to as microparticulated tuberculin (or its synonym).

### Example 1: Suppressive effect of tumor vaccine of the present invention against growth of liver cancer

### A. Materials and methods

### 1. Preparation of immunoadjuvant

1) A 25% human serum albumin solution (HSA, Baxter Albumac, Baxter) was diluted with sterilized water to a concentration of 2.5% and adjusted to pH 2.5 with 4 N HCl.
2) The resulting 2.5% human serum albumin solution and a heparin solution (Novo Heparin 1000, 1000 U/ml, about 7.69 mg/ml or less, Aventis Pharma Ltd.) were mixed in various proportions beforehand to determine the optimal mixing ratio of the heparin solution. Microparticles were initially prepared at an arbitrary mixing ratio and centrifuged at 2,500 rpm (1,300 g) for 15 minutes, and the protein content in the supernatant was quantified by using Protein Assay Kit 1 (Japan Bio-Rad Laboratories, Inc., Tokyo). The optimal mixing ratio was defined as a ratio providing a condition under which not less than 99.9% of the mixed proteins were taken into the microparticles.
3) Tuberculin (Japan BCG Manufacturing Co., Ltd., purified tuberculin for general diagnosis (tuberculin purified protein derivative, PPD) for one person, amount equivalent to 0.25 *µ*g of a standard product) was suspended in a heparin solution for injection and added dropwise with a 2.5% human serum albumin solution at a given ratio with stirring by using a vortex mixer.
4) The mixture was centrifuged at 4,300 rpm (1,300 g) for 15 minutes, and the precipitates were washed twice with sterilized distilled water and resuspended in sterilized distilled water.
5) The suspension was added with 1-ethyl-3-(3-dimethylaminopropyl)carbodiimide solution (EDC, SIGMA, dissolved in water at 20 mg/ml) at a final concentration of 0.8 to 1.5 mg/ml with stirring by using a vortex mixer and then left standing at room temperature for 15 minutes.
6) The mixture was centrifuged at 4,300 rpm (1,300 g) for 15 minutes, and the precipitates were washed 6 times with sterilized distilled water and resuspended in sterilized physiological saline at a required concentration to obtain a microparticulated tuberculin suspension.

### 2. Measurement of suppressive effect of tumor vaccine against growth of mouse liver cancer

1) Hepa 1-6 liver cancer cells, cultured until they became confluent in a plastic culture dish having a diameter of 10 cm, were immobilized with a 3% paraformaldehyde solution at room temperature for 2 hours. The cells were washed with Dulbecco's phosphate buffered saline containing calcium and magnesium (PBS(+)), further washed and sterilized with 70% ethanol, and thoroughly washed again with PBS(+).
2) These cells were added with 10 ml per dish of DMEM medium and incubated at 37°C for 2 days.
3) The cells were thoroughly washed with PBS(+), added with 3 ml per dish of a poly-L-lysine solution (25 u g/ml) and incubated for 2 hours.
4) The cells were thoroughly washed with PBS(+), and then the total immobilized cells were scraped off with a scraper and suspended in PBS(+).
5) In a volume of 200 µl of the suspension corresponding to the cells of 4 dishes was placed in an Eppendorf tube, added with 100 µl of a tuberculin solution (0.25 µg/100 µl PBS(+)) and incubated for 2 hours.
6) The mixture was added with 150 *µ*l of the microparticulated tuberculin suspension (containing tuberculin at a concentration of 3 *µ*g/ml), and further added with 50 *µ*l of PBS(+) to obtain a microparticulated tuberculin vaccine. In the same manner, 200 *µ*l of a solution obtained by diluting 5 KE/ml OK432 (Chugai Pharmaceutical Co., Ltd., Picibanil 5KE) 10 times with PBS(+) was placed in an Eppendorf tube instead of the microparticulated tuberculin and PBS(+) to obtain a vaccine for the positive control group.
7) Nine mice syngeneic with the Hepa 1-6 liver cancer cells (C57L/J, 6- to 9-week old, males) were intracutaneously injected with 50 *µ*l per animal of the above microparticulated tuberculin vaccine. This procedure was repeated one week later. Nine mice in the control group was injected with only Dulbecco's phosphate buffered saline not containing calcium or magnesium (PBS(-)). Further, the animals of the positive control group was injected with the vaccine for the positive control group prepared in the above 6) in a similar manner.
8) Further one week later, the animals were subcutaneously injected with Hepa 1-6 cells (1 x 10⁷ cells/0.2 ml PBS(-)) at the right legs.
9) After the above challenge, the animals were intracutaneously injected with 50 µl per animal of a microparticulated tuberculin vaccine prepared in the same manner up to the above step 6) (provided that the microparticulated tuberculin used for preparation of this vaccine contained tuberculin at a concentration of 2 *µ*g/ml). The 9 mice in the control group was injected only with PBS(-). Further, the mice in the positive control group were injected with the vaccine for the positive control group prepared in the above 6) in a similar manner.
10) Then, survival rate of the mice was determined after growth of subcutaneous liver cancer.

### B. Results

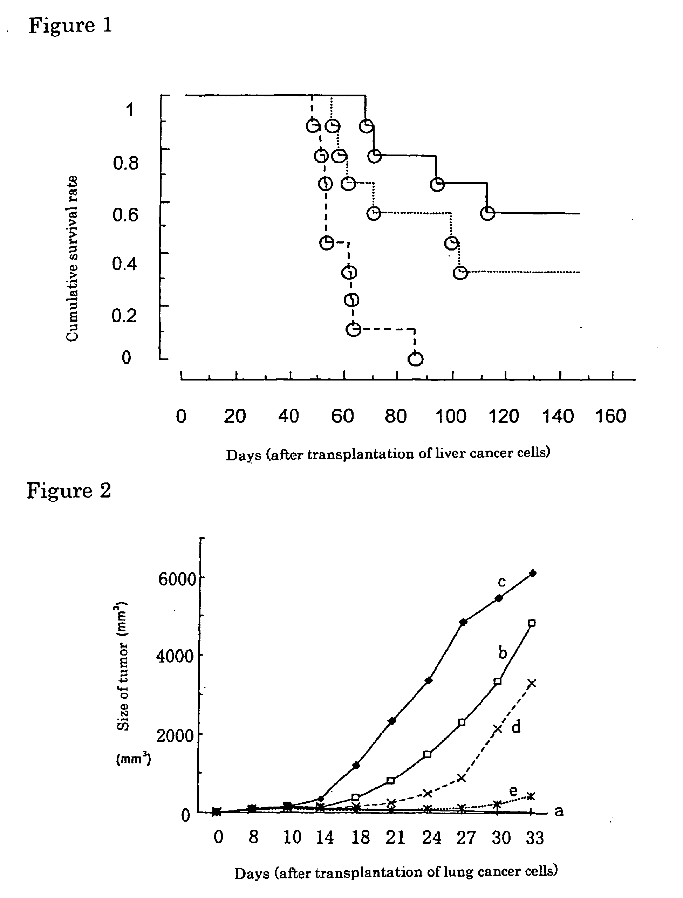

The results are shown in Fig. 1. This figure shows Kaplan-Meier curves for the control group, the group administered with the microparticulated tuberculin vaccine, and the positive control group. The group administered with the microparticulated tuberculin vaccine showed a survival rate higher than that of the control group with a statistically significant difference (log-rank test, p<0.0001). Moreover, the administered group also showed antitumor effect superior to that observed in the positive control group.

### Example 2: Tumor recurrence prevention effect of microparticulated tuberculin administered into tumor tissue coagulated with microwave

### A. Materials and methods

1. Microparticulated tuberculin was prepared in the same manner as Step 1 of Example 1.
2. C57BL/6 mice (females, 6 animals per group) were intracutaneously injected with 1 x 10⁶ of syngenic lung cancer cells (Lewis lung carcinoma cell strain) at the right legs, and when the size of the subcutaneous lung cancer tissue reached about 75 mm³ on the 8th day, the mice were anesthetized with pentobarbital sodium. Then, the skin aside of the lung cancer tissue was cut, and Microtaze endoscopy-use mono-ball type electrode (E-24N, diameter: 2.4 mm) was inserted into the cancer tissue from the section.
3. The electrode was connected to Microtaze (Model HSE-8M, Azwell Inc.), and the tissue was irradiated with microwaves at 10 W for 3 minutes until the cancer tissue apparently gave complete coagulation by heating.
4. The wound was sutured, and a tuberculin solution (obtained by dissolving 25 ng of purified tuberculin in 25 *µ*l of physiological saline) and suspended microparticulated tuberculin (containing 50 ng of purified tuberculin, suspended in 5 *µ*l of physiological saline) were injected into the cancer tissue on the 10th day. On the 14th day, this injection was repeated again (group a). The mice in the control group (group b) were subjected to only the microwave irradiation. Further, a control group not irradiated with microwave (group c), a group administered with OK-432 (1 KE/25 µl) instead of the microparticulated tuberculin (group d), and a group administered with OK-432 (1 KE/25 µl) instead of the tuberculin solution (group e) were also prepared.
5. Then, changes in the size (mm³) of cancer tissue enlarged due to recurrence were measured over time.

### B. Results

The results are shown in Fig. 2. Each point shows an average value of the size of lung cancer tissue in 6 mice. In the observation up to the 33rd day after the transplantation of the lung cancer cells, recurrence of lung cancer in the tissues was clearly suppressed in the group a in comparison with the groups b and c. In particular, no recurrence was observed in any of 6 animals in the group a receiving the microparticulated tuberculin and the tuberculin solution, and thus the group gave complete suppression. Whilst, in the group d not receiving the microparticulated tuberculin and the group e not receiving the tuberculin solution, the cancer tissues once shrank after the microwave irradiation and grew again, and each of these was a small cancer tissue. Although the cancer proliferation was suppressed compared with the groups b and c, recurrence was observed in 5 animals among 6 animals in these groups (Table 1).

**Table 1**

| Recurrence after transplantation of lung cancer cells | |
|---|---|
| Number of mice in which lung cancer recurred among 6 animals per group | |
| a: Microwave irradiation + tuberculin solution + microparticulated tuberculin | 0 |
| b: Microwave irradiation | 5 |
| c: No microwave irradiation | 6 |
| d: Microwave irradiation + OK-432 + tuberculin solution | 5 |
| e: Microwave irradiation + OK-432 + microparticulated tuberculin | 5 |

### Industrial Applicability

The immunoadjuvant of the present invention can efficiently exhibit potent adjuvant activity and avoid conditions undesirable for living bodies.

## Claims

1. An immunoadjuvant for administration to a tumor tissue denatured by a physical means, which comprises:
precipitates formed by coacervation of
(a) a soluble protein, provided that a soluble protein contained in tuberculin is excluded, and
(b) a mucopolysaccharide
and further comprises:
(c) a soluble protein contained in tuberculin
wherein said (c) is coprecipitated with the precipitates.

2. The immunoadjuvant according to claim 1, wherein the physical means is selected from a group consisting of microwave irradiation, radio frequency coagulation, freezing coagulation, electrosurgical knife heating, hot water injection, alcohol injection, embolization, radioactive ray irradiation, laser beam irradiation, and ultrasonic disruption.

3. A tumor vaccine comprising the immunoadjuvant according to claim 1 or 2.

## Patentansprüche

1. Ein Immunoadjuvans zur Verabreichung in ein Tumorgewebe, das durch physikalische Mittel denaturiert wurde, wobei das Immunoadjuvans Folgendes umfasst:
Präzipitate, die durch Koazervierung der folgenden Substanzen gebildet wurden:
(a) ein lösliches Protein, unter der Maßgabe, dass ein in Tuberkulin enthaltenes lösliches Protein ausgeschlossen ist; und
(b) ein Mucopolysaccharid;
und wobei das Immunoadjuvans weiterhin Folgendes umfasst:
(c) ein lösliches Protein, das in Tuberkulin enthalten ist, wobei das Protein aus (c) mit den Präzipitaten kopräzipitiert.

2. Das Immunoadjuvans gemäß Anspruch 1, **dadurch gekennzeichnet, dass** das physikalische Mittel ausgewählt ist aus der Gruppe MikrowellenBestrahlung, Hochfrequenz-Koagulation, Gefrier-Koagulation, Erhitzen mit einem Elektrochirurgie-Messer, Heißwasser-Injektion, Alkohol-Injektion, Embolisierung (Blutgefäßverschluss), Bestrahlung mit radioaktiver Strahlung, Laserstrahl-Bestrahlung und Ultraschall-Zerreißung.

3. Ein Tumorvakzin enthaltend das Immunoadjuvans gemäß Anspruch 1 oder 2.

## Revendications

1. Immunoadjuvant pour l'administration à un tissu tumoral dénaturé par un moyen physique, qui comprend :
des précipités formés par coacervation de
(a)une protéine soluble, à condition que soit exclue une protéine soluble contenue dans la tuberculine, et
(b)un mucopolysaccharide
et qui comprend de plus :
(c)une protéine soluble contenue dans la tuberculine
dans lequel ladite (c) est coprécipitée avec les précipités.

2. Immunoadjuvant selon la revendication 1, dans lequel le moyen physique est choisi dans le groupe formé par l'irradiation par micro-ondes, la coagulation à haute fréquence, la cryocoagulation, le chauffage au bistouri électrique, l'injection d'eau chaude, l'injection d'alcool, l'embolisation, l'irradiation par rayons radioactifs, l'irradiation laser et la lyse ultrasonore.

3. Vaccin antitumoral comprenant l'immunoadjuvant selon la revendication 1 ou 2.
